(19) **Europäisches Patentamt / European Patent Office / Office européen des brevets**

(11) **EP 1 340 487 B1**

(12) **EUROPEAN PATENT SPECIFICATION**

(45) Date of publication and mention of the grant of the patent:
**09.06.2010 Bulletin 2010/23**

(51) Int Cl.:
*A61K 8/35* *(2006.01)*     *A61K 8/39* *(2006.01)*
*A61K 8/362* *(2006.01)*     *A61K 8/365* *(2006.01)*
*A61K 8/46* *(2006.01)*     *A61K 8/49* *(2006.01)*
*A61Q 5/02* *(2006.01)*

(21) Application number: **03004335.0**

(22) Date of filing: **28.02.2003**

(54) **Hair cleaning compositions comprising a dicarboxylic acid and/or hydroxydicarboxylic acid**

Haarwaschmittel enthaltend Dicarbonsäuren und/oder Hydroxydicarbonsäuren

Compositions de shampooing comprenant des acides dicarboxyliques et/ou des hydroxyacides dicarboxyliques

(84) Designated Contracting States:
**DE FR GB**

(30) Priority: **01.03.2002 JP 2002055885**

(43) Date of publication of application:
**03.09.2003 Bulletin 2003/36**

(73) Proprietor: **KAO CORPORATION**
**Tokyo 103-8210 (JP)**

(72) Inventor: **Terazaki, Hiroyuki,**
**Kao Corporation**
**Sumida-ku,**
**Tokyo 131-8501 (JP)**

(74) Representative: **HOFFMANN EITLE**
**Patent- und Rechtsanwälte**
**Arabellastrasse 4**
**81925 München (DE)**

(56) References cited:
**EP-A- 0 855 178     EP-A- 1 016 401**
**DE-A- 2 341 785     US-A- 5 785 962**
**US-B1- 6 231 843**

- **PATENT ABSTRACTS OF JAPAN vol. 2002, no. 05, 3 May 2002 (2002-05-03) -& JP 2002 029940 A (KAO CORP), 29 January 2002 (2002-01-29)**
- **PATENT ABSTRACTS OF JAPAN vol. 2000, no. 14, 5 March 2001 (2001-03-05) -& JP 2000 327540 A (KAO CORP), 28 November 2000 (2000-11-28)**
- **M.W. HELIOFF, K. SCOTT MARSI: "Shampoo Innovation via a new Surfactant" DRUG & COSMETIC INDUSTRY, vol. 142, no. 4, 1988, pages 38-42,**

Remarks:
The file contains technical information submitted after the application was filed and not included in this specification

## Description

### Technical Field

This invention relates to hair cleansing compositions, which have good foamability and high-lubricity foam quality upon washing, and are excellent in the luster, softness and manageability of hair after drying and also superb in stability.

### Background Art

In hair treatments such as hair rinses and hair conditioners, it is practiced to lower the pHs of their systems to impart luster and softness to hair. No attempt has, however, been made to lower the pHs of hair cleansing compositions, because the hair cleansing compositions are intended to impart neither luster nor softness in general.

With a view to providing hair cleansing compositions with functions to give such luster, softness and manageability as described above, the present inventors conducted research on low-pH hair cleansing compositions which made use of organic acids. A problem, however, arose in that in a low pH range, anionic surfactants as cleansing ingredients underwent decomposition, resulting in stability deteriorations such as gelling of systems and reductions in foamability. On the other hand, hair itself has a buffer capacity. A simple application of a treatment to hair, therefore, leads to a change in the pH of the treatment, raising a problem that its intended effects cannot be fully brought about.

### Disclosure of the Invention

The present invention has as an object the provision of a hair cleansing composition, which is excellent in stability, can effectively impart luster, softness and manageability to hair, and has good foamability and high-lubricity foam quality upon washing.

The present inventors have found that impartment of a buffer capacity to a hair cleansing composition can inhibit decomposition of an anionic surfactant in a low pH range and can also reduce a change in pH during hair washing, and as a result, that a hair cleansing composition capable of meeting the above-described requirements can be obtained.

Described specifically, the present invention provides a hair cleansing composition comprising the following ingredients (A) to (C):

(A) 0,05 to 10% by weight of a dicarboxylic acid or hydroxydicarboxylic acid or a salt thereof,
(B) 0,01 to 50% by weight of an organic solvent selected from the following organic solvents (b2) to (b4):

(b2) an N-alkylpyrrolidone with an alkyl group, which has 1 to 18 carbon atoms, bonded to the nitrogen atom,
(b3) an alkylene carbonate having 1 to 4 carbon atoms,
(b4) a polypropylene glycol having a number-average molecular weight of from 200 to 5,000, and

(C) 1 to 50% by weight of an anionic surfactant having a sulfate group;

wherein the ranges of amounts each are based on the whole composition;
wherein the hair cleansing composition has a pH of from 1 to 4 when diluted 20-fold by weight with water, and also a buffer capacity not lower than 0.005 gram equivalent/L but lower than 0.2 gram equivalent/L.

Owing to the above-described features, the hair cleansing composition according to the present invention has good foamability and high-lubricity foam quality upon washing and is excellent in the luster, softness and manageability of hair after drying and also superb in stability.

### Best Modes for Carrying out the Invention

Examples of the dicarboxylic acid as the ingredient (A) can include malonic acid, succinic acid, glutaric acid, adipic acid, maleic acid, fumaric acid, phthalic acid, and oxalic acid, with maleic acid being particularly preferred. Examples of the hydroxydicarboxylic acid as the ingredient (A) can include malic acid and tartaric acide, with malic acid being particularly preferred. Examples of the salt of the dicarboxylic acid or hydroxydicarboxylic acid can include its sodium salt, potassium salt, and ammonium salt.

Two or more of these dicarboxylic acids, hydroxydicarboxylic acids and salts thereof may be used in combination as the ingredient (A). From the standpoint of improvements in the finish of hair such as luster and manageability, the content of the ingredient (A) ranges from 0.05 to 10 wt.%, preferably from 0.1 to 5 wt.%, particularly from 0.5 to 2 wt.% in terms of free acid, all based on the hair cleansing composition according to the present invention.

Among the organic solvents as the ingredient (B), illustrative of the N-alkylpyrrolidone (b2) are N-methylpyrrolidone, N-octylpyrrolidone, and N-laurylpyrrolidone. Illustrative of the alkylene carbonate (b3) are ethylene carbonate and propylene carbonate. Preferred examples of the polypropylene glycol (b4) can include those having molecular weights of from 200 t0 1,000, especially those having molecular weights of from 300 to 400.

Two or more of these organic solvents may be used in combination as the ingredient (B). From the standpoint of improvements in use feeling, luster and softness, the content of the ingredient (B) ranges from 0.01 to 50 wt.%, preferably from 0.1 to 35 wt.%, particularly from 0.5 to 10 wt.%, all based on the hair cleansing composition according to the present invention.

Examples of the sulfate-type anionic surfactant as the ingredient (C) can include polyoxyethylene alkyl ether sulfates, polyoxyethylene alkenyl ether sulfates, alkyl sulfates, and polyoxyalkylene alkyl phenyl ether sulfates. Particularly preferred are those represented by the following formula (5) or (6):

$$R^6O(CH_2CH_2O)_mSO_3M \qquad (5)$$

$$R^7OSO_3M \qquad (6)$$

wherein $R^6$ represents an alkyl group or alkenyl group having 10 to 18 carbon atoms, $R^7$ represents an alkyl group having 10 to 18 carbon atoms, M represents an alkali metal, alkaline earth metal, ammonium, alkanolamine or basic amino acid, and m stands for a number of from 1 to 5 in terms of weight average.

Two or more of these sulfates may be used in combination as the ingredient (C). From the standpoint of foamability and also of liquid properties and cleansing property at the time of use, the content of the ingredient (C) ranges from 1 to 50 wt.%, preferably from 8 to 30 wt.%, particularly from 10 to 22 wt.%, all based on the hair cleansing composition according to the present invention.

To improve the finish after drying, one or more conditioning ingredients selected from cationic polymers, cationic surfactants, silicones and oils may be incorporated further in the hair cleansing composition according to the present invention.

Examples of the cationic polymers can include cationic cellulose derivatives, cationic starch, cationic guar gum derivatives, homopolymer of diallyl quaternary ammonium salt, diallyl quaternary ammonium salt/acrylamide copolymer, quaternized polyvinylpyrrolidone derivatives, polyglycol polyamine condensates, vinylimidazolium trichloride/vinylpyrrolidone copolymer, hydroxyethylcellulose/dimethyldiallylammonium chloride copolymer, vinylpyrrolidone/quaternized dimethylaminoethyl methacrylate copolymer, polyvinylpyrrolidone/alkyl aminoacrylate copolymer, polyvinylpyrrolidone/alkyl aminoacrylate/vinyl caprolactam copolymers, vinylpyrrolidone/methacrylamidopropyl trimethylammonium chloride copolymer, alkylacrylamide/ acrylate/alkylaminoalkylacrylamide/polyethylene glycol methacrylate copolymers, adipic acid/ dimethylaminohydroxypropyl ethylenetriamine copolymer ("Cartaretin"; product of Sandoz Chemicals Corp., U.S.A.), and cationic polymers disclosed in JP-A-53139734 and JP-A-60036407. Particularly preferred are cationic cellulose derivatives and cationic guar gum derivatives.

Example of the cationic surfactants can include lauryltrimethylammonium chloride, cetyltrimethylammonium chloride, cetyltrimethylammonium bromide, stearyltrimethylammonium chloride, stearyltrimethylammonium bromide, lauryltrimethylammonium bromide, dialkyldimethylammonium chlorides, dicetyldimethylammonium chloride, distearyldimethylammonium chloride, dicocoyldimethylammnoium chloride, myristyldimethylbenzylammnoium chloride, stearyldimethylbenzylammonium chloride, lanolin fatty acid aminopropylethyldimethylammonium ethyl sulfate, lanolin fatty acid aminoethyltriethylammonium ethyl sulfate, stearylamidopropyldimethylamine (and its salts), stearylamidoethyldiethylamine (and its salts), lanolin fatty acid aminopropyltriethylammonium ethyl sulfate, lanolin fatty acid aminoethyltrimethylammonium methyl sulfate, lanolin fatty acid aminopropylethyldimethylammonium methyl sulfate, isoalkanoaic acid $(C_{14}-C_{20})$ aminopropylethyldimethylammonium ethyl sulfate, isoalkanoic acid $(C_{18}-C_{22})$ aminopropylethyldimethylammonium ethyl sulfate, isostearic acid aminopropylethyldimethylammonium ethyl sulfate, isononanoic acid aminopropylethyldimethyl ammonium ethyl sulfate, and alkyltrimethylammonium saccharins.

Examples of the silicones can include the followings:

(Silicones-1) Dimethylpolysiloxanes
Illustrative are those represented by the following formula:

$$(Me)_3SiO-[(Me)_2SiO]_d-Si(Me)_3$$

wherein each Me represents a methyl group, and d stands for a number of from 3 to 20,000.
(Silicones-2) Amino-modified silicones
One having an average molecular weight of from about 3,000 to 100,000 and listed under the name of "Amodimethicone" in the third edition of the CTFA dictionary (Cosmetic Ingredient Dictionary, U.S.A.) is preferred, although a variety of amino-modified silicones are usable. This amino-modified silicone can be used preferably as an aqueous emulsion, and its commercial products include "SM 8704C" (Dow Corning Toray Silicone Co., Ltd.) and "DC 929" (Dow Corning Corporation).

(Silicones-3) Other silicones

As silicones other than those described above, there are also polyether-modified silicones, methylphenylpolysiloxane, fatty-acid-modified silicones, alcohol-modified silicones, alkoxy-modified silicones, epoxy-modified silicones, fluorine-modified silicones, cyclic silicones, alkyl-modified silicones, and the like.

The term "oils" as used herein means oils other than silicones. Illustrative are hydrocarbons such as squalene, squalane, liquid paraffin, liquid isoparaffin, and cycloparaffin; glycerides such as castor oil, cacao oil, mink oil, avocado oil, and olive oil; waxes such as beeswax, whale wax, lanolin, and carnauba wax; alcohols such as cetyl alcohol, oleyl alcohol, stearyl alcohol, isostearyl alcohol, 2-octyldodecanol, and glycerin; esters such as isopropyl palmitate, isopropyl myristate, octyldodecyl myristate, hexyl laurate, cetyl lactate, propylene glycol monostearate, oleyl oleate, hexadecyl 2-ethylhexanoate, isononyl isononanoate, and tridecyl isononanoate; higher fatty acids such as capric acid, lauric acid, myristic acid, palmitic acid, stearic acid, behenic acid, oleic acid, coconut oil fatty acids, isostearic acid, and isopalmitic acid; isostearyl glyceryl ethers; and polyoxypropyleen butyl ether. Of these, esters are preferred, with hexadecyl 2-ethylhexanoate, isononyl isononanoate and isopropyl palmitate are particularly preferred.

Two or more of these conditioning ingredients may be used in combination. From the standpoint of foam lubricity and also of smoothness from washing to rinsing, the content of the conditioning ingredient may range preferably from 0.05 to 10 wt.%, more preferably from 0.1 to 5 wt.%, particularly from 0.3 to 2 wt.%, all based on the hair cleansing composition according to the present invention.

To further improve the foamability of the hair cleansing composition according to the present invention, one or more surfactants other than the ingredient (C), said surfactants being selected from anionic surfactants, nonionic surfactants and amphoteric surfactants, may also be incorporated.

The anionic surfactants other than the ingredient (C) can include sulfonate-type anionic surfactants and carboxylate-type anionic surfactants. Illustrative are alkyl sulfosuccinate salts, alkyl polyoxyalkylene sulfosuccinate salts, higher fatty acid salts, and alkanesulfonate salts.

Examples of the nonionic surfactants can include polyoxyalkylene sorbitan fatty acid esters, polyoxyalkylene sorbitol fatty acid esters, polyoxyalkylene glycerol fatty acid esters, polyoxyalkylene fatty acid esters, polyoxyalkylene alkyl ethers, polyoxyalkylene alkyl phenyl ethers, polyoxyalkylene (hydrogenated) castor oils, sucrose fatty acid esters, polyglyceryl alkyl ethers, polyglyceryl fatty acid esters, fatty acid alkanolamides, and alkyl glycosides. Among these, alkyl glycosides, polyoxyalkylene ($C_8$-$C_{20}$) fatty acid esters, polyoxyethylene sorbitan fatty acid esters, polyoxyethylene hydrogenated castor oil and fatty acid alkanolamides are preferred. Preferred fatty acid alkanolamides are those containing acyl groups having the carbon numbers of from 8 to 18, especially from 10 to 16. The fatty acid alkanolamides can be either monoalkanolamides or dialkanolamides. Preferred are those containing hydroxyalkyl groups having the carbon numbers of from 2 to 3. Illustrative are oleic diethanolamide, palm kernel oil fatty acid diethanolamide, coconut oil fatty acid diethanolamide, lauric diethanolamide, polyoxyethylene coconut oil fatty acid monoethanolamides, coconut oil fatty acid monoethanolamides, lauric isopropanolamide, and lauric monoethanolamide.

The amphoteric surfactants can include betaine-type surfactants. Among these, betaine-type surfactants such as alkyldimethylaminoacetic acid betaines and fatty acid amidopropyl betaines are more preferred, with fatty acid amidopropyl betaines being particularly preferred. Of these fatty acid amidopropyl betaines, preferred are those having acyl groups whose carbon numbers are from 8 to 18, especially from 10 to 16. Particularly preferred are lauramidopropyl betaine, palm kernel oil fatty acid amidopropyl betaines, and coconut oil fatty acid amidopropyl betaines.

In addition to the above-described ingredients, ingredients which are employed in ordinary hair cleansing compositions can also be incorporated in the hair cleansing composition according to the present invention as needed depending upon the purpose of use. Such ingredients can include, for example, antidandruff agents; vitamins; germicides; anti-inflammatories; chelating agents; humectants such as sorbitol and panthenol; colorants such as dyes and pigments; viscosity controlling agents such as hydroxyethylcellulose, methylcellulose, polyethylene glycol, ethanol, and clay mineral; pH adjusters such as potassium hydroxide; plant extracts; pearlants; fragrances; color additives; ultraviolet absorbers; antioxidants; and ingredients described in ENCYCLOPEDIA OF SHAMPOO INGREDIENTS (MICELLE PRESS).

From the viewpoint of imparting luster and softness to hair and also reducing irritation, the hair cleansing composition according to the present invention has a pH of from 1 to 4, preferably from 2 to 4, particularly from 3 to 3.9 when diluted 20-fold by weight with water (to the concentration upon application to hair).

From the standpoint of inhibiting decomposition of the ingredient (C) and also reducing changes in pH during hair cleaning, the hair cleansing composition according to the present invention is required to have a buffer capacity not lower than 0.005 gram equivalent/L but lower than 0.2 gram equivalent/L, preferably not lower than 0.01 gram equivalent/L but lower than 0.2 gram equivalent/L, more preferably not lower than 0.015 gram equivalent/L but lower than 0.2 gram equivalent/L when diluted 20-fold by weight with water. The term "buffer capacity" as used herein means a value determined by the following equation while using as a measure the concentration of a base required to raise the pH of the 20-fold aqueous solution at 25°C by 1 from its initial value.

$$\texttt{Buffer capacity = }|dC_B/dpH|$$

wherein $C_B$ represents an ion concentration (gram equivalent/L) of the base.

Such a buffer capacity can be imparted by adding a pH buffering agent or the like to the hair cleansing composition. Usable as the pH buffering agent is an organic acid or inorganic acid or a salt thereof, which has buffering action in a pH range of from 1 to 4. Examples of the organic acid can include, in addition to the ingredient (A), citric acid, glycolic acid, lactic acid, levulinic acid, butyric acid, valeric acid, and mandelic acid. Examples of the inorganic acid can include phosphoric acid, sulfuric acid, and nitric acid. Further, examples of the salt of such an acid can include its alkali metal salts such as the sodium salt and the potassium salt; its ammonium salt; and its alkanolamine salts such as the triethanolamine salt. No particular limitation is imposed on the amount of the pH buffering agent to be added, and its amount varies depending on the kind of the compound giving buffering ability. When sodium citrate is used as a primary compound giving the buffering ability, for example, it can be added at a concentration of about 1 wt.% or higher.

The form of the hair cleansing composition according to the present invention can be chosen as desired, including a liquid form, a powder form, a gel form, and a granular form. However, a liquid form making use of water or a lower alcohol as a solvent, especially water is preferred.

The hair cleansing composition according to the present invention can be formulated into one for use in a bathroom a shampoo composition, a shampoo with rinse, a treatment or a conditioner, especially a shampoo composition.

Examples

In the following Examples and Comparative Examples, each "pH" indicates a pH as measured when diluted 20-fold by weight with water.

Examples 1-4 and Comparative Examples 1-4

Shampoo compositions shown in Table 1 were prepared, and their organoleptic ranking was conducted.

(Washing method)

Subsequent to thorough moistening of hair, 5 g or 10 g (5 g for semi-long hair, 10 g for long hair) of a shampoo composition were dispensed, and then, the hair was washed. The hair was rinsed thoroughly and then dried fully with hot air from a dryer.

(Organoleptic ranking)

Ranking was conducted by five expert panelists in accordance with the following ranking systems, and based on average scores, the shampoo compositions were ranked.

- Ranking systems

(1) Luster of hair after drying

4: Pronounced improvement in luster is observed.
3: Improvement in luster is observed.
2: Some improvement in luster is observed.
1: No improvement in luster.
0: Luster is lost.

(2) Softness of hair after drying

4: Very soft.

3: Soft.
2: Slightly soft.
1: Not soft.
0: Not soft at all.

(3) Manageability of hair after drying

4: Very good manageability.
3: Good manageability.
2: Average manageability.
1: Somewhat poor manageability.
0: No manageability.

- Ranking

A: Average ranking score ≥ 3.5
B: 3.5 > Average ranking score ≥ 2.5
C: 2.5 > Average ranking score ≥ 1.5
D: 1.5 > Average ranking score

Table 1

| Ingredients (wt.%) | | Examples | | | | Comparative Examples | | | |
|---|---|---|---|---|---|---|---|---|---|
| | | 1 | 2 | 3 | 4 | 1 | 2 | 3 | 4 |
| (A) | Maleic acid | 1 | - | - | - | - | - | - | - |
| | Malic acid | - | 1 | 1 | 1 | - | - | - | 0.05 |
| (B) | Polypropylene glycol (Mw = 400) | 1 | - | - | 0.5 | 1 | - | - | - |
| | Propylene carbonate | - | 1 | - | - | - | - | - | - |
| | N-methylpyrrolidone | - | - | 0.5 | - | - | 0.5 | - | - |
| (C) | Sodium POE(2) lauryl ether sulfate | 10 | 10 | 10 | 10 | 10 | 10 | 10 | 10 |
| | Sodium lauryl sulfate | 5 | 5 | 5 | 5 | 5 | 5 | 5 | 5 |
| Others | Myristyl alcohol | 1 | 1 | 1 | 1 | 1 | 1 | 1 | 1 |
| | Cocoyl monoethanolamide | 0.5 | 0.5 | 0.5 | 0.5 | 0.5 | 0.5 | 0.5 | 0.5 |
| | Ethylene glycol distearate | 1 | 1 | 1 | 1 | 1 | 1 | 1 | 1 |
| | Cationic hydroxyethylcellulose | 0.3 | 0.3 | 0.3 | 0.3 | 0.3 | 0.3 | 0.3 | 0.3 |
| | Cationic guar gum | 0.5 | 0.5 | 0.5 | 0.5 | 0.5 | 0.5 | 0.5 | 0.5 |
| | NaOH Aqueous solution | q.s.* | q.s.* | q.s.* | q.s.* | q.s.* | q.s.* | q.s.* | q.s.* |
| | Purified water | Bal. | Bal. | Bal. | Bal. | Bal. | Bal. | Bal. | Bal. |
| pH (when diluted 20-fold by weight) | | 3.5 | 3.5 | 3.5 | 3.5 | 3.5 | 3.5 | 3.5 | 5.0 |
| Buffer capacity (NaOH-gram equivalent/L) | | 0.02 | 0.01 | 0.01 | 0.01 | 0.003 | 0.003 | 0.003 | 0.002 |

(continued)

| Ingredients (wt.%) | | Examples | | | | Comparative Examples | | | |
|---|---|---|---|---|---|---|---|---|---|
| | | 1 | 2 | 3 | 4 | 1 | 2 | 3 | 4 |
| Ranking | Luster of hair | A | A | A | A | C | B | B | B |
| | Manageability of hair | A | A | A | A | B | C | B | B |
| | Softness of hair | A | A | A | A | C | C | C | C |

* Amount sufficient to adjust the pH.

Example 5 Clear Shampoo

|  | (wt.%) |
|---|---|
| Sodium POE(2) lauryl ether sulfate | 10.0 |
| Cationic guar gum | 0.1 |
| Malic acid | 0.75 |
| Sodium chloride | 1.0 |
| Lauramidopropyl betaine | 1.0 |
| Cocoyl monoethanolamide | 0.3 |
| Propylene carbonate | 0.5 |
| Glycerin | 1.0 |
| Sodium hydroxide | q.s. to pH 4 |
| Deionized water | Balance |

The above-described shampoo (pH: 4.0, buffer capacity: 0.008) was excellent in foam volume and foam lubricity upon washing, smoothness upon rinsing, and hair luster and softness after drying, and was also superb in stability.

Example 6 Conditioning Shampoo

|  | (wt.%) |
|---|---|
| Sodium POE(2) lauryl ether sulfate | 8.0 |
| Cationic guar gum | 0.5 |
| Maleic acid | 0.75 |
| Disodium citrate | 1.0 |
| Lauramidopropyl betaine | 3.0 |
| Cocoyl monoethanolamide | 0.7 |
| Myristyl alcohol | 1.0 |
| Ethylene glycol distearate | 3.0 |
| Polypropylene glycol (Mw = 400) | 0.5 |
| Glycerin | 1.0 |
| Deionized water | Balance |

The above-described shampoo (pH: 3.5, buffer capacity: 0.02) was excellent in foam volume and foam lubricity upon washing, smoothness upon rinsing, and hair luster and softness after drying, and was also superb in stability.

Example 7 Conditioning Shampoo

|  | (wt.%) |
|---|---|
| Sodium POE(2) lauryl ether sulfate | 11.0 |
| Sodium lauryl sulfate | 5.0 |
| Cationic guar gum | 0.3 |
| Malic acid | 0.75 |
| Lactic acid | 0.1 |
| Sodium chloride | 0.2 |
| Benzyl alcohol | 0.5 |
| N-methylpyrrolidone | 0.2 |
| Propylene carbonate | 0.3 |
| Cocoyl monoethanolamide | 1.0 |
| Dimethicone (viscosity: 100,000 mPa·s) | 0.5 |
| Amodimethicone | 0.1 |
| Myristyl alcohol | 1.0 |
| Cetanol | 0.5 |
| Ethylene glycol distearate | 3.0 |
| Cationic hydroxyethylcellulose | 0.3 |
| Glycerin | 1.0 |
| Sodium hydroxide | q.s. to pH 3.7 |
| Deionized water | Balance |

The above-described shampoo (pH: 3.7, buffer capacity: 0.009) was excellent in foam volume and foam lubricity upon washing, smoothness upon rinsing, and hair luster and softness after drying, and was also superb in stability.

Example 8 Clear Shampoo

|  | (wt.%) |
|---|---|
| Sodium POE(2) lauryl ether sulfate | 10.0 |
| Cationic cellulose | 0.1 |
| Malic acid | 0.75 |
| Sodium chloride | 1.0 |
| Lauramidopropyl betaine | 1.0 |
| Cocoyl monoethanolamide | 0.3 |
| Propylene carbonate | 0.5 |
| Glycerin | 1.0 |
| Sodium hydroxide | q.s. to pH 4 |
| Deionized water | Balance |

The above-described shampoo (pH: 4.0, buffer capacity: 0.008) was excellent in foam volume and foam lubricity upon washing, smoothness upon rinsing, and hair luster and softness after drying, and was also superb in stability.

**Claims**

1. A hair cleansing composition comprising the following ingredients (A) to (C):

(A) 0.05 to 10% by weight of a dicarboxylic acid or hydroxydicarboxylic acid or a salt thereof,
(B) 0.01 to 50% by weight of an organic solvent selected from the following organic solvents (b2) to (b4):

(b2) an N-alkylpyrrolidone with an alkyl group, which has 1 to 18 carbon atoms, bonded to said nitrogen atom,
(b3) an alkylene carbonate having 1 to 4 carbon atoms,

(b4) a polypropylene glycol having a number-average molecular weight of from 200 to 5,000, and

(C) 1 to 50% by weight of an anionic surfactant having a sulfate group;

wherein the ranges of amounts each are based on the whole composition; wherein said hair cleansing composition has a pH of from 1 to 4 when diluted 20-fold by weight with water, and also a buffer capacity not lower than 0.005 gram equivalent/L but lower than 0.2 gram equivalent/L.

**2.** A hair cleansing composition according to claim 1, further comprising as an ingredient (D) at least one conditioning ingredient selected from cationic polymers, cationic surfactants, silicones and oils.

**Patentansprüche**

**1.** 1. Haarreinigungszusammensetzung, umfassend die folgenden Bestandteile (A) bis (C):

(A) 0,05 bis 10 Gew.-% einer Dicarbonsäure oder einer Hydroxydicarbonsäure oder eines Salzes davon,
(B) 0,01 bis 50 Gew.-% eines organischen Lösungsmittels, ausgewählt aus den folgenden organischen Lösungsmitteln (b2) bis (b4):

(b2) N-Alkylpyrrolidon mit einer Alkylgruppe, die 1 bis 18 Kohlenstoffatome hat, die an das Stickstoffatom gebunden ist,
(b3) Alkylencarbonat mit 1 bis 4 Kohlenstoffatomen,
(b4) Polypropylenglycol mit einem Molekulargewicht im Zahlenmittel von 200 bis 5.000, und

(C) 1 bis 50 Gew.-% eines anionischen Tensides mit einer Sulfatgruppe;

worin die Mengenbereiche jeweils auf die gesamte Zusammensetzung bezogen sind; worin die Haarreinigungszusammensetzung einen pH von 1 bis 4 aufweist, wenn sie auf das 20-fache des Gewichtes mit Wasser verdünnt ist, und ebenfalls eine Pufferkapazität von nicht weniger als 0,005 Gramäquivalent/l, aber weniger als 0,2 Gramäquivalent/l hat.

**2.** Haarreinigungszusammensetzung nach Anspruch 1, weiterhin umfassend als Bestandteil (D) zumindest einen Konditionierbestandteil, ausgewählt aus kationischen Polymeren, kationischen Tensiden, Silikonen und Ölen.

**Revendications**

**1.** Composition nettoyante pour les cheveux comprenant les ingrédients (A) à (C) suivants :

(A) 0,05 à 10 % en poids d'un acide dicarboxylique ou d'un acide hydroxydicarboxylique ou d'un sel de celui-ci,
(B) 0,01 à 50 % en poids d'un solvant organique choisi parmi les solvants organiques (b2) à (b4) suivants :

(b2) une N-alkylpyrrolidone avec un groupe alkyle, qui possède 1 à 18 atomes de carbone, lié audit atome d'azote,
(b3) un carbonate d'alkylène possédant 1 à 4 atomes de carbone,
(b4) un polypropylèneglycol ayant un poids moléculaire moyen en nombre de 200 à 5 000, et

(C) 1 à 50 % en poids d'un surfactant anionique ayant un groupe sulfate ;

dans laquelle les gammes de quantités sont basées chacune sur la composition entière ; dans laquelle ladite composition nettoyante pour les cheveux a un pH de 1 à 4 lorsqu'elle est diluée 20 fois en poids avec de l'eau, et également une capacité tampon non inférieure à 0,005 équivalent-gramme/l mais inférieure à 0,2 équivalent-gramme/l.

**2.** Composition nettoyante pour les cheveux selon la revendication 1, comprenant en outre comme ingrédient (D) au moins un ingrédient traitant choisi parmi les polymères cationiques, les surfactants cationiques, les silicones et les huiles.

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 53139734 A
- JP 60036407 A